(19) Europäisches Patentamt
European Patent Office
Office européen des brevets

(11) Numéro de publication: **0 093 049**
**B1**

# (12) FASCICULE DE BREVET EUROPEEN

(45) Date de publication du fascicule du brevet: **16.07.86**

(51) Int. Cl.⁴: **C 07 D 519/00,** A 61 K 31/495 // (C07D519/00, 495:00, 471:00)

(21) Numéro de dépôt: **83400793.2**

(22) Date de dépôt: **21.04.83**

(54) **Nouveaux dérivés du dithiinno (1,4)(2,3-c)pyrrole, leur préparation et les médicaments qui les contiennent.**

(30) Priorité: **22.04.82 FR 8206943**

(43) Date de publication de la demande: **02.11.83 Bulletin 83/44**

(45) Mention de la délivrance du brevet: **16.07.86 Bulletin 86/29**

(84) Etats contractants désignés: **AT BE CH DE FR GB IT LI LU NL SE**

(56) Documents cités: **US - A - 3 948 917**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Farge, Daniel, 30, rue des Pins Sylvestres, F-94320 Thiais (FR)**
Inventeur: **Leger, André, 97, rue des Morillons, F-75015 Paris (FR)**
Inventeur: **Ponsinet, Gérard, 7, rue de Grand Champ, F-94370 Sucy-en-Brie (FR)**

(74) Mandataire: **Le Goff, Yves et al, RHONE-POULENC RECHERCHES Brevets Pharma 25, Quai Paul Doumer, F-92408 Courbevoie (FR)**

**EP 0 093 049 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du dithiinno [1,4][2,3-c]pyrrole de formule générale:

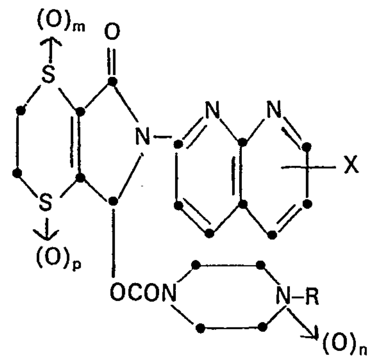

(I)

leurs sels, leur préparation et les compositions médicinales qui les contiennent.

Dans la formule générale (I), X représente un atome d'halogène, R représente un radical alcoyle contenant 1 à 4 atomes de carbone en chaîne droit ou ramifiée, n, m et p représentent des nombres entiers égaux à 0 ou 1, étant entendu que m est égal à 0 lorsque p est égal à 1 et que m est égal à 1 lorsque p est égal à 0 et que les produits de formule générale (I) se présentent sous la forme cis pure ou en mélange.

On entend par forme cis la configuration stéréochimique telle que l'atome d'oxygène du sulfoxyde et le radical alcoylpipérazinylcarbonyloxy sont situés du même côté du plan de la molécule formé par le cycle dithiinno[1,4][2,3-c] pyrrole.

Selon l'invention, les produits de formule générale (I) dans laquelle les différents symboles sont définis comme précédemment peuvent être obtenus par oxydation du produit de formule générale (II):

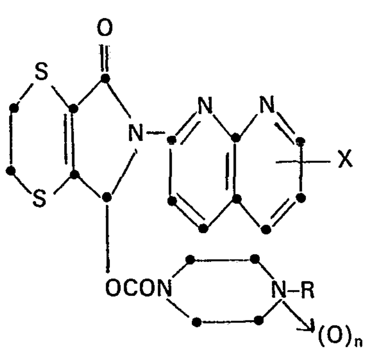

(II)

dans laquelle n est un nombre entier égal à 0 ou 1, c'est-à-dire a la définition correspondante, suivie d'une séparation des produits obtenus.

L'oxydation peut être réalisée en employant environ un équivalent d'un agent couramment utilisé pour passer d'un sulfure à un sulfoxyde, en opérant dans un solvant approprié. Par exemple, on peut employer l'eau oxygénée dans l'acétone ou l'acide acétique, un périodate alcalin dans un alcool ou l'acétonitrile, un peroxyacide carboxylique (acide paracétique, perbenzoïque, m-chloroperbenzoïque, p-nitroperbenzoïque ou perphthalique) dans un éther (dioxanne, tétrahydrofuranne, oxyde de diéthyle), un solvant chloré (dichlorométhane, dichloroéthane), l'acide acétique ou un mélange de ces solvants. La réaction s'effectue généralement à une température comprise entre −10 et +20 °C.

Il est particulièrement avantageux d'opérer dans un mélange d'acide acétique et de dichlorométhane en présence d'acide m-chloroperbenzoïque, à une température comprise entre −10 et 0 °C.

Selon une variante de l'invention, les produits de formule générale (I) dans laquelle n est égal à 1 et les autres symboles sont définis comme précédemment peuvent aussi être obtenus par oxydation d'un produit de formule générale:

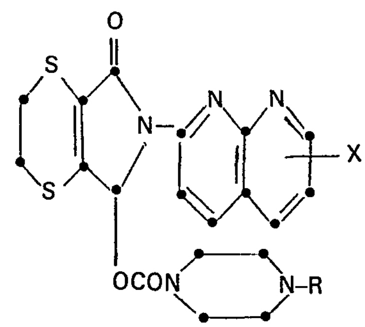

(III)

dans laquelle R et X sont définis comme précédemment. Dans ce cas, on opère en présence d'au moins deux équivalents d'un agent couramment utilisé pour oxyder à la fois un sulfure et une amine, en opérant dans un solvant approprié. Par exemple, on peut employer l'eau oxygénée dans l'acétone ou l'acide acétique ou un peroxyacide carboxylique (acide peracétique, perbenzoïque, m-chloroperbenzoïque, p-nitroperbenzoïque, perphthalique) dans un éther (dioxanne, tétrahydrofuranne, oxyde de diéthyle), un solvant chloré (dichlorométhane, dichloroéthane), l'acide acétique ou un mélange de ces solvants. La réaction s'effectue généralement à une température comprise entre −10 et +20 °C.

Il est particulièrement avantageux d'opérer dans le dichlorométhane en présence d'un excès d'acide m-chloroperbenzoïque, à une température comprise entre −10 et 0 °C.

La séparation des différents produits d'oxydation peut être effectuée par tout moyen physique ou chimique habituel à la portée de l'homme du métier. Il est particulièrement avantageux d'effectuer la séparation par chromatographie.

Les produits de formules générales (II) et (III) peuvent être préparés comme décrit dans le brevet américain 3 948 917.

Les nouveaux produits selon l'invention peuvent être transformés en sels d'addition avec les acides. Ces sels peuvent être obtenus par action des nouveaux dérivés sur les acides dans des solvants appropriés; comme solvants, on peut utiliser des alcools, des éthers, des cétones ou des solvants chlorés; le sel formé précipite après concentration éventuelle de sa solution et est séparé par filtration ou décantation.

Sont d'un intérêt particulier:

– le (chloro-7-naphthyridine-[1,8] yl-2)-6-[(méthyl-4-pipérazinyl-1) carbonyloxy] -5-oxo-7 tétrahydro-2, 3,6,7 5H-dithiinno[1,4] [2,3-c] pyrrole-oxyde-4 cis

– le (chloro-7 naphthyridine [1,8] yl-2)-6 [(méthyl-4 pipérazinyl-1) carbonyloxy] -5 oxo-7 tétrahydro-2,3,6,7 5H-dithiinno [1,4][2,3-c] pyrrole-oxyde-1, mélange cis + trans

– le {[chloro-7 naphthyridine [1,8] yl-2)-6 oxo-7 tétrahydro-2,3,6,7 5H-dithiinno [1,4] [2,3-c] pyrrolyl-5 oxyde-1] oxycarbonyl}-4 méthyl-1 pipérazine-oxyde-1, mélange cis + trans.

Les produits de formules générales (II) et (III) ont une action tranquillisante, anticonvulsivante et hypnogène. Il a maintenant été trouvé que les nouveaux produits selon l'invention, c'est-à-dire les monosulfoxydes correspondants, possèdent une activité tranquillisante et anticonvulsivante voisine de celle des produits de formules générales (II) et (III) mais présentent des propriétés «hypnogènes» plus faibles susceptibles de les rendre plus aptes au traitement de certaines maladies (état anxieux, épilepsie, ...).

L'activité tranquillisante des produits selon l'invention peut être mise en évidence chez l'animal (souris) à des doses comprises entre 1 et 10 mg/kg par voie orale dans le test des convulsions induites par le pentétrazol selon une technique voisine de celle de EVERETT et RICHARDS [J. Pharmacol. Exp. Ther. 81, 402, 1944)].

Chez la souris, par voie orale, dans le test de potentialisation d'une faible dose de chlorpropazine mesurée dans le test de Righting Reflex [selon ZBINDEN et RANDALL, Advances in Pharmacology, 2, 213–291 (1967)] (test permettant de prévoir les effets «hypnogènes» d'un produit), les produits selon l'invention ne se sont montrés actifs qu'à des doses supérieures à 30 mg/kg.

De plus, les produits selon l'invention présentent une faible toxicité. Chez la souris, la toxicité aiguë (exprimée par sa $DL_{50}$) est généralement comprise entre 300 et 900 mg/kg ou même supérieure à 900 mg/kg.

Les exemples suivants, donnés à titre non limitatif, montrent comment l'invention peut être mise en pratique.

Exemple 1

A une solution refroidie à −10 °C de 47,3 g de (chloro-7 naphthyridin [1,8] yl-2)-6 [(méthyl-4 pipérazinyl-1) carbonyloxy]-5 oxo-7 tétrahydro-2,3,6,7 5H-dithiinno [1,4][2,3-c] pyrrole dans 750 $cm^3$ de dichlorométhane et 150 $cm^3$ d'acide acétique, on ajoute sous agitation une solution de 24 g d'acide chloro-3 perbenzoïque à 80% dans 500 $cm^3$ de dichlorométhane. L'addition est effectuée goutte à goutte en 15 minutes, de façon à maintenir la température entre −10 et −5 °C. Après 30 minutes d'agitation supplémentaire, on ajoute 3 litres d'oxyde de diéthyle puis on filtre pour séparer un produit solide A et les liqueurs-mères B.

Le solide A est lavé avec 200 $cm^3$ d'oxyde de diéthyle et séché sous pression réduite (0,2 mm de mercure; 0,027 kPa) à 20 °C. Le produit obtenu est chromatographié sur 300 g de gel de gel de silice (0,04–0,06 mm) contenus dans une colonne de 6 cm de diamètre. On élue par un mélange de 4500 $cm^3$ de chloroforme et 300 $cm^3$ de méthanol puis par un mélange de 1500 $cm^3$ de chloroforme et 150 $cm^3$ de méthanol en recueillant des fractions de 100 $cm^3$. On réunit les fractions 9 à 15 et évapore le solvant sous pression réduite (20 mm de mercure, 2,7 kPa) à 40 °C. Le résidu est dissous dans un mélange de 140 $cm^3$ de dichlorométhane et 20 $cm^3$ de méthanol, additionné de noir décolorant et filtré. On ajoute au filtrat 200 $cm^3$ d'oxyde de diéthyle. Les cristaux qui se forment sont séparés par filtration et séchés sous pression réduite (0,2 mm de mercure, 0,027 kPa) à 40 °C. On obtient ainsi 9,3 g de (chloro-7 naphthyridin [1,8]yl-2)-6 [(méthyl-4-pipérazinyl-1) carbonyloxy]-5 oxo-7 tétrahydro-2,3,6,7 5H-dithiinno [1,4][2,3-c] pyrrole-oxyde-1, mélange de diastéréoisomères cis et trans fondant avec décomposition vers 255 °C.

Les fractions 21 à 39 de la chromatographie ci-dessus sont réunies, évaporées sous pression réduite (20 mm de mercure; 2,7 kPa) à 40 °C. Le résidu est chromatographié sur 100 g de gel de silice (0,04–0,06 mm) contenus dans une colonne de 4 cm de diamètre. On élue par un mélange de 1000 $cm^3$ de chloroforme et 800 $cm^3$ de méthanol en recueillant des fractions de 50 $cm^3$. On réunit les fractions 12 et 13, évapore le solvant sous pression réduite (20 mm de mercure; 2,7 kPa) à 40 °C et sèche le résidu sous pression réduite (0,2 mm de mercure; 0,027 kPa) à 60 °C. On obtient ainsi 0,94 g de [chloro-7 naphthyridin [1,8]yl-2)-6 [(méthyl-4 pipérazinyl-1) carbonyloxy]-5 oxo-7 tétrahydro-2,3,6,7 6H-dithiinno [1,4][2,3-c] pyrrole oxyde-4 cis fondant avec décomposition vers 250 °C.

Exemple 2

A une solution refroidie à −10 °C de 19,12 g de (chloro-7 naphthyridin [1,8]yl-2)-6 [(méthyl-4 pipérazinyl-1) carbonyloxy]-5 oxo-7 tétrahydro-2,3,6,7 5H-dithiinno [1,4] [2,3-c] pyrrole dans 250 $cm^3$ de dichlorométhane, on ajoute une solution de 16,69 g d'acide chloro-3 perbenzoïque à 80% dans 150 $cm^3$ de chlorure de méthylène. L'addition est effectuée goutte à goutte en 30 minutes de façon à maintenir la température entre −10 °C et −5 °C. On agite encore pendant deux heures à cette température puis filtre en recueillant le filtrat dans 800 $cm^3$ d'oxyde de diéthyle. Le précipité obtenu est séparé par filtration, lavé à l'oxyde de diéthyle et séché à l'air. On reprend ce produit dans 700 $cm^3$ d'éthanol bouillant contenant 2 g de noir décolorant, filtre à chaud et refroidit à 0 °C. Les cristaux formés sont séparés par filtration, lavés avec 50 $cm^3$ d'éthanol et séchés sous pression réduite (0,2 mm de mercure; 0,027 kPa) à 60 °C. On obtient ainsi 15 g de

chloro-3 benzoate de [(chloro-7 naphthyridin [1,8]yl-2)-6 oxo-7 tétrahydro-2,3,6,7 5H-dithiinno [1,4] [2,3-c] pyrrolyl-5 oxyde-1] oxycarbonyl -4 méthyl-1 pipérazine-oxyde-1, mélange de diastéréoisomères cis et trans fondant avec décomposition vers 190 °C.

Pour l'emploi médicinal, il est fait usage des nouveaux composés soit à l'état de bases, soit à l'état de sels pharmaceutiquement acceptables, c'est-à-dire non toxiques aux doses d'utilisation.

Comme exemples de sels d'addition pharmaceutiquement acceptables peuvent être cités des sels d'acides minéraux (tels que les chlorhydrates, sulfates, nitrates, phosphates) ou organiques (tels que les acétates, propionates, succinates, benzoates, fumarates, maléates, tartrates, théophylline-acétates, salicylates, phénolphthalinates, méthylène bis-β-oxynaphthoates ou des dérivés de substitution de ces acides.

La présente invention concerne les médicaments constitués par les produits de formule générale (I), sous forme libre ou sous forme de sel d'addition avec un acide pharmaceutiquement acceptable, à l'état pur ou en association avec tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, disperants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits selon l'invention sont particulièrement utiles dans le traitement de certaines maladies se manifestant par des états anxieux ou épileptiformes. Les doses dépendent de l'effet recherché et de la durée du traitement; elles sont généralement comprises entre 0,2 et 50 mg par jour par voie orale pour un adulte en une ou plusieurs prises.

D'une façon générale, le médecin déterminera la posologie qu'il estime la plus appropriée en fonction de l'âge, du poids et des tous les autres facteurs propres au sujet à traiter.

L'exemple suivant, donné à titre non limitatif, illustre des compositions selon l'invention.

Exemple

On prépare selon la technique habituelle, des comprimés dosés à 10 mg de produit actif ayant la composition suivante:

| | |
|---|---|
| – (chloro-7 naphthyridin [1,8] yl-2)-6 [(méthyl-4 pipérazinyl-1) carbonyloxy]-5 oxo-7 tétrahydro-2,3,6,7 5H-dithiinno [1,4] [2,3-c] pyrrole-oxyde-1, mélange de diastéréoisomères cis et trans | ... 10 mg |
| – amidon | ... 60 mg |
| – lactose | ... 50 mg |
| – stéarate de magnésium | ... 2 mg |

**Revendications**

pour les Etats contractants BE, CH, DE, FR, GB, IT, LI, LU, NL, SE

1. Un nouveau dérivé du dithiinno [1,4] [2,3-c] pyrrole caractérisé en ce qu'il répond à la formule générale:

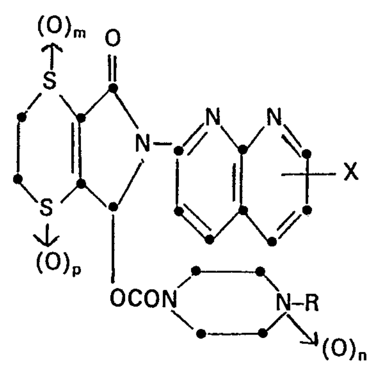

dans laquelle X représente un atome d'halogène, R représente un radical alcoyle contenant 1 à 4 atomes de carbone, n, m et p représentent des nombres entiers égaux à 0 ou 1, étant entendu que m est égal à 0 lorsque p est égal à 1 et que m est égal à 1 lorsque p est égal à 0 et que le produit

se présente sous la forme cis pure ou en mélange, ainsi que ses sels d'addition avec les acides.

2. Un procédé de préparation d'un produit selon la revendication 1 caractérisé en ce que l'on oxyde un sulfure de formule générale:

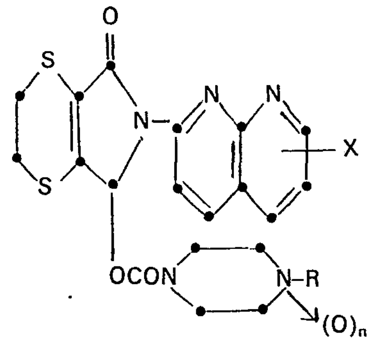

dans laquelle n, R et X sont définis comme dans la revendication 1 puis isole le produit obtenu.

3. Un procédé selon la revendication 2 caractérisé en ce que l'oxydation est effectuée au moyen de tout agent d'oxydation connu pour oxyder un sulfure organique en sulfoxyde correspondant.

4. Un procédé selon la revendication 3 caractérisé en ce que l'agent d'oxydation est choisi dans le groupe constitué par l'eau oxygénée, les périodates alcalins, les peroxyacides carboxyliques et utilisé à raison d'environ un équivalent par mole de produit à oxyder.

5. Un procédé de préparation d'un produit selon la revendication 1 dans la formule duquel n est égal à 1 et m, p, R et X sont définis comme dans la revendication 1, caractérisé en ce que l'on oxyde un produit de formule générale:

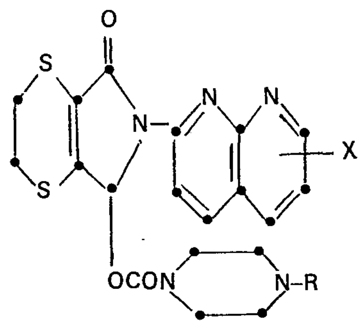

puis isole le produit attendu.

6. Un procédé selon la revendication 5 caractérisé en ce que l'oxydation est effectuée au moyen de tout agent d'oxydation connu pour oxyder un sulfure organique en sulfoxyde correspondant et une amine en N-oxyde correspondant.

7. Un procédé selon la revendication 6 caractérisé en ce que l'agent d'oxydation est choisi dans le groupe constitué par l'eau oxygénée et les peroxyacides carboxyliques et utilisé à raison d'au moins 2 équivalents par mole de produit à oxyder.

8. Un médicament caractérisé en ce qu'il contient un produit selon la revendication 1 en association avec un ou plusieurs diluants ou adjuvants compatibles et pharmaceutiquement acceptables.

**Revendications**

pour l'Etat contractant AT

1. Procédé de préparation d'un nouveau dérivé du dithiinno [1,4] [2,3-c) pyrrole de formule générale:

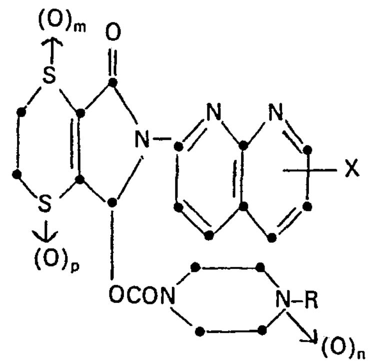

dans laquelle X représente un atome d'halogène, R représente un radical alcoyle contenant 1 à 4 atomes de carbone, n, m et p représentent des nombres entiers égaux à 0 ou 1, étant entendu que m est égal à 0 lorsque p est égal à 1 et que m est égal à 1 lorsque p est égal à 0 et que le produit se présente sous la forme cis pure ou en mélange, ainsi que ses sels d'addition avec les acides, caractérisé en ce que:

a) l'on oxyde un sulfure de formule générale:

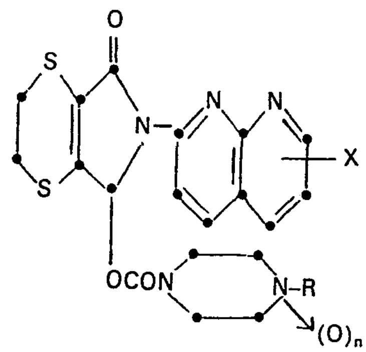

dans laquelle n, R et X sont définis comme précédemment puis sépare et isole les produits obtenus ou en ce que:

b) l'on oxyde un produit de formule générale:

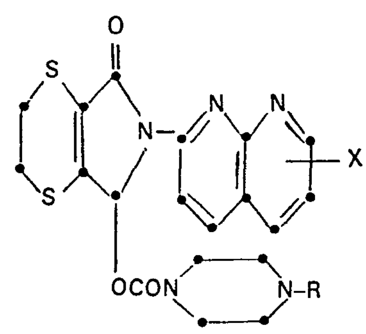

dans laquelle R et X sont définis comme précédemment pour obtenir le produit correspondant dans la formule duquel n représente un nombre entier égal à 1 et les autres symboles sont définis

comme précédemment, puis sépare et isole les produits attendus.

2. Un procédé selon la revendication 1-a) caractérisé en ce que l'oxydation est effectuée au moyen de tout agent d'oxydation connu pour oxyder un sulfure organique en sulfoxyde correspondant.

3. Un procédé selon la revendication 2 caractérisé en ce que l'agent d'oxydation est chosi dans le groupe constitué par l'eau oxygénée, les périodates alcalins, les peroxyacides carboxyliques et utilise à raison d'environ un équivalent par mole de produit à oxyder.

4. Un procédé selon la revendication 1-b) caractérisé en ce que l'oxydation est effectuée au moyen de tout agent d'oxydation connu pour oxyder un sulfure organique en sulfoxyde correspondant et une amine en N-oxyde correspondant.

5. Un procédé selon la revendication 4 caractérisé en ce que l'agent d'oxydation est choisi dans le groupe constitué par l'eau oxygénée et les peroxyacides carboxyliques et utilisé à raison d'au moins 2 équivalents par mole de produit à oxyder.

**Patentansprüche**
für die Vertragsstaaten BE, CH, DE, FR, GB, IT, LI, LU, NL, SE

1. Ein neues Derivat des Dithiinno [1,4] [2,3-c] pyrrols, dadurch gekennzeichnet, dass es der allgemeinen Formel

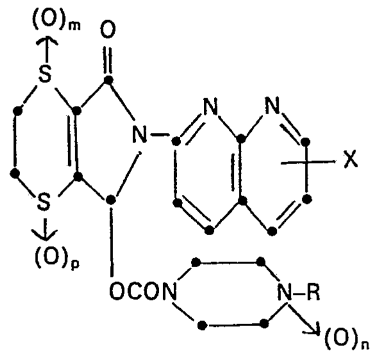

entspricht, worin X ein Halogenatom bedeutet, R einen Alkylrest mit 1 bis 4 Kohlenstoffatomen bedeutet, n, m und p ganze Zahlen gleich 0 oder 1 bedeuten, wobei m gleich 0 ist, wenn p = 1 und wobei m gleich 1 ist, wenn p = 0 und dass das Produkt in reiner cis-Form oder im Gemisch vorliegt, sowie seine Additionssalze mit Säuren.

2. Verfahren zur Herstellung eines Produkts gemäss Anspruch 1, dadurch gekennzeichnet, dass man ein Sulfid der allgemeinen Formel

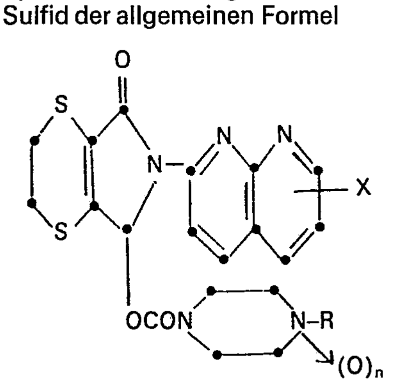

worin n, R und X wie in Anspruch 1 definiert sind, oxidiert und das erhaltene Produkt dann isoliert.

3. Verfahren gemäss Anspruch 2, dadurch gekennzeichnet, dass die Oxidation mit jedem Oxidationsmittel, das zum Oxidieren eines organischen Sulfids zum entsprechenden Sulfoxid bekannt ist, bewirkt wird.

4. Verfahren gemäss Anspruch 3 dadurch gekennzeichnet, dass das Oxidationsmittel ausgewählt ist aus der Gruppe bestehend aus Wasserstoffperoxid, Alkaliperiodaten, den Peroxicarbonsäuren und in einer Menge von etwa einem Äquivalent pro Mol zu oxidierendes Produkt verwendet wird.

5. Verfahren zur Herstellung eines Produkts gemäss Anspruch 1, in dessen Formel n = 1 und m, p, R und X wie in Anspruch 1 definiert sind, dadurch gekennzeichnet, dass man ein Produkt der allgemeinen Formel

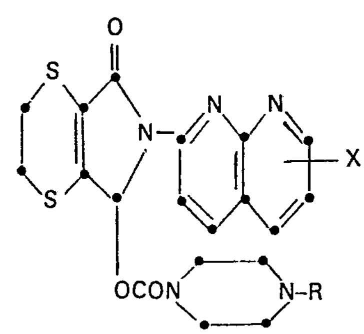

oxidiert und das erwartete Produkt dann isoliert.

6. Verfahren gemäss Anspruch 5, dadurch gekennzeichnet, dass die Oxidation mit jedem Oxidationsmittel, das zum Oxidieren eines organischen Sulfids zum entsprechenden Sulfoxid und eines Amins zum entsprechenden N-Oxid bekannt ist.

7. Verfahren gemäss Anspruch 6, dadurch gekennzeichnet, dass das Oxidationsmittel ausgewählt ist aus der Gruppe bestehend aus Wasserstoffperoxid, den Peroxicarbonsäuren und in einer Menge von wenigstens zwei Äquivalenten pro Mol zu oxidierendes Produkt verwendet wird.

8. Arzneimittel, dadurch gekennzeichnet, dass es ein Produkt gemäss Anspruch 1 zusammen mit einem oder mehreren verträglichen und pharmazeutisch annehmbaren Verdünnungs- oder Hilfsmitteln enthält.

**Patentansprüche**
für den Vertragsstaat AT

1. Verfahren zur Herstellung eines neuen Derivats des [1,4]-Dithiinno-[2,3-c]-pyrrols der allgemeinen Formel:

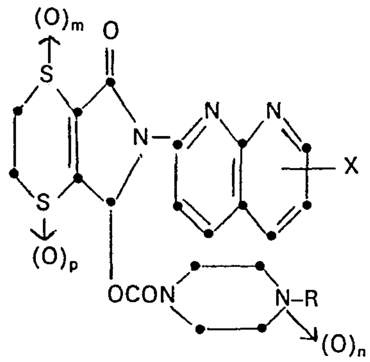

in welcher X ein Halogenatom darstellt, R für einen Alkylrest mit 1 bis 4 Kohlenstoffatomen steht, n, m und p ganze Zahlen gleich 0 oder 1 darstellen, wobei selbstverständlich m gleich 0 ist, wenn p gleich 1 ist, und m gleich 1 ist, wenn p gleich 0 ist, und die Verbindung in reiner cis-Form oder als Mischung vorliegt, sowie von deren Säureadditionssalzen, dadurch gekennzeichnet, dass man

a) ein Sulfid der allgemeinen Formel:

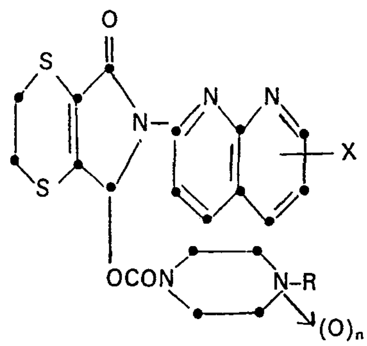

worin n, R und X die obige Bedeutung haben, oxidiert und dann die erhaltenen Verbindungen trennt und isoliert oder dass man

b) eine Verbindung der allgemeinen Formel:

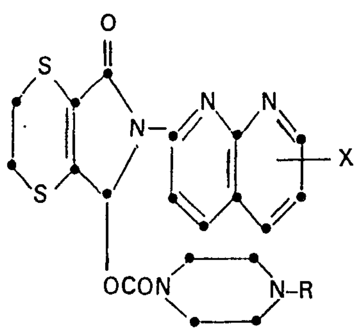

in welcher R und X die obige Bedeutung haben, unter Bildung der entsprechenden Verbindung, in deren Formel n eine ganze Zahl gleich 1 darstellt und die übrigen Symbole die obige Bedeutung haben, oxidiert und dann die erwarteten Produkte trennt und isoliert.

2. Verfahren nach Anspruch 1-a), dadurch gekennzeichnet, dass die Oxidation mittels irgendeines bekannten Oxidationsmittels für die Oxidation eines organischen Sulfids zum entsprechenden Sulfoxid ausgeführt wird.

3. Verfahren nach Anspruch 2, dadurch gekennzeichnet, dass das Oxidationsmittel ausgewählt ist aus der Gruppe, bestehend aus Wasserstoffperoxid, den Alkaliperjodaten und den Peroxycarbonsäuren, und in einer Menge von etwa einem Äquivalent pro Mol zu oxidierender Verbindung eingesetzt wird.

4. Verfahren nach Anspruch 1-b), dadurch gekennzeichnet, dass die Oxidation mittels irgendeines bekannten Oxidationsmittels für die Oxidation eines organischen Sulfids zum entsprechenden Sulfoxid und eines Amins zum entsprechenden N-Oxid ausgeführt wird.

5. Verfahren nach Anspruch 4, dadurch gekennzeichnet, dass das Oxidationsmittel ausgewählt ist aus der Gruppe, bestehend aus Wasserstoffperoxid und den Peroxycarbonsäuren, und in einer Menge von zumindest 2 Äquivalenten pro Mol zu oxidierender Verbindung eingesetzt wird.

## Claims

for the Contracting States BE, CH, DE, FR, GB, IT, LI, LU, NL, SE

1. New dithiino [1,4][2,3-c]pyrrole derivative, characterised in that it corresponds to the general formula:

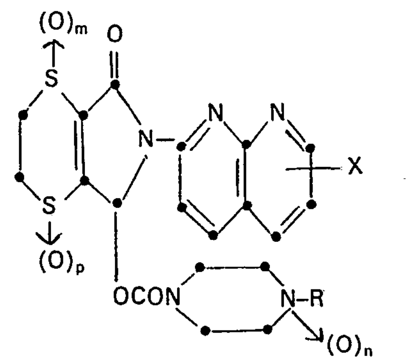

in which X denotes a halogen atom, R denotes an alkyl radical containing 1 to 4, carbon atoms, n, m and p denote integers equal to 0 or 1, on the understanding that m equals 0 when p equals 1 and that m equals 1 when p equals 0, and that the product takes the cis form, pure or mixed, as well as its addition salts with acids.

2. Process for preparing a product according to Claim 1, characterised in that a sulphide of general formula:

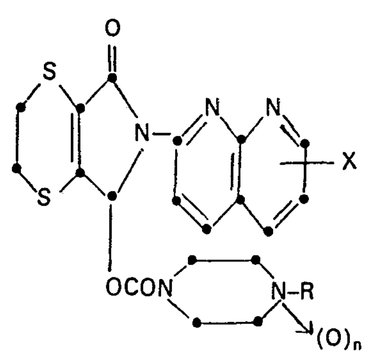

in which n, R and X are defined as in Claim 1, is

oxidised, and the product obtained is then isolated.

3. Process according to Claim 2, characterised in that the oxidation is performed using any oxidising agent known to oxidise an organic sulphide to the corresponding sulphoxide.

4. Process according to Claim 3, characterised in that the oxidising agent is chosen from the group consisiting of hydrogen peroxide, alkali metal periodates and carboxylic peroxy acids, and is used in the proportion of approximately one equivalent per mole of product to be oxidised.

5. Process for preparing a product according to Claim 1 in the formula of which n equals 1 and m, p, R and X are defined as in Claim 1, characterised in that a product of general formula:

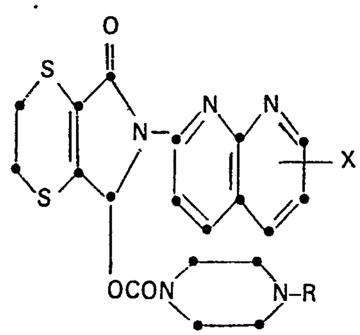

is oxidised, and the expected product is then isolated.

6. Process according to Claim 5, characterised in that the oxidation is peroformed using any oxidising agent known to oxidise an organic sulphide to the corresponding sulphoxide and an amine to the corresponding N-oxide.

7. Process according to Claim 6, characterised in that the oxidising agent is chosen from the group consisting of hydrogen peroxide and carboxylic peroxy acids, and is used in the proportion of at least 2 equivalents per mole of product to be oxidised.

8. Drug, characterised in that it contains a product according to Claim 1 in combination with one or more diluents or adjuvants which are compatible and pharmaceutically acceptable.

## Claims

for the Contracting State AT

1. Process for preparing a new dithiino [1,4][2,3-c] -pyrrole derivative of general formula:

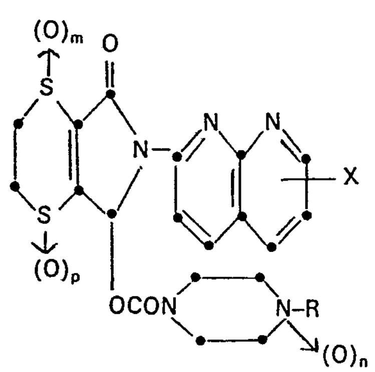

in which X denotes a halogen atom, R denotes an alkyl radical containing 1 to 4, carabon atoms, n, m and p denote integers equal to 0 or 1, on the understanding that m equals 0 when p equals 1 and that m equals 1 when p equals 0, and that the product takes the cis form, pure or mixed, as well as its addition salts with acids, characterised in that:

a) a sulphife of general formula:

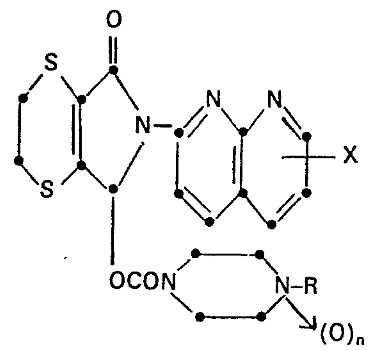

in which n, R and X are defined as above, is oxidised, and the products obtained are then separated and isolated, or in that:

b) a product of general formula:

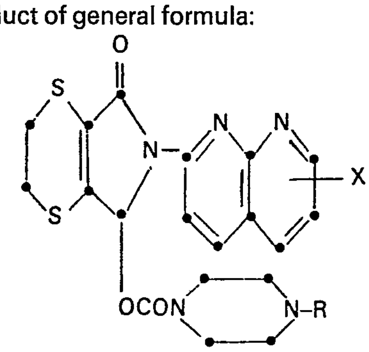

in which R and X are defined as above, is oxidised to obtain the corresponding product in the formula of which n denotes an integer equal to 1 and the other symbols are defined as above, and the expected products are then separated and isolated.

2. Process according to Claim 1-a), characterised in that the oxidation is performed using any oxidising agent known to oxidise an organic sulphide ti the corresponding sulphoxide.

3. Process according to Claim 2, characterised in that the oxidising agent is chosen from the group consisting of hydrogen peroxide, alkali metal periodates and carboxylic peroxy acids, and is used in the proportion of approximately one equivalent per mole of product to be oxidised.

4. Process according to Claim 1-b), characterised in that the oxidation is performed using any oxidising agent known to oxidise an organic sulphide to the corresponding sulphoxide and an amine to the corresponding N-oxide.

5. Process according to Claim 4, characterised in that the oxidising agent is chosen from the group consisting of hydrogen peroxide and carboxylic peroxy acids, and is used in the proportion of at least 2 equivalents per mole of product to be oxidised.